# EUROPÄISCHE PATENTSCHRIFT

(11) **EP 1 136 047 B1**
(45) Veröffentlichungstag und Bekanntmachung des Hinweises auf die Patenterteilung: **01.09.2004**
(21) Anmeldenummer: 01810186.5
(22) Anmeldetag: 22.02.2001
(51) Int. Cl.: A61F 2/42

(54) **Künstliches Fingergelenk**
Artificial finger joint
Articulation artifivielle du doigt

(30) Priorität: 21.03.2000 EP 00810236
(43) Veröffentlichungstag der Anmeldung: 26.09.2001
(73) Patentinhaber: Centerpulse Orthopedics Ltd., 6340 Baar (CH)
(72) Erfinder: Rauscher, Markus, 6319 Allenwinden (CH)
(74) Vertreter: Manitz, Finsterwald & Partner Gbr

(56) Entgegenhaltungen:
- DE-A- 4 337 922
- US-A- 5 674 297

## Beschreibung

Die Erfindung handelt von einem künstlichen Fingergelenk mit einem konvexen Gelenkkopf und mit einer konkaven Gelenkschale, die unabhängig voneinander jeweils mit einem Schaft in einem Knochenende befestigbar sind und in einer Artikulationsebene aus einer Extensionsstellung mit parallelen Schaftachsen in eine Hyperextensionsstellung oder in eine Artikulationsendstellung bewegbar sind. Dokument US-A-5 674 297 stellt den nächsten Stand der Technik dar.

Künstliche Fingergelenke kommen zwischen Mittelhandknochen und Fingerknochen oder zwischen einzelnen Fingerknochen vor. Um an einem solchen Fingergelenk operieren zu können, muss der Operateur die Knochenenden freilegen und minutiös Blutgefässe, Nerven und Sehnen seitlich wegschieben, ohne dass diese überdehnt werden. Es ist daher üblich mit dem seitlichen Wegschieben, welches eine Dehnung verursachen würde, das Gelenk in Artikulationsstellung zu bringen, in welcher beide Knochenenden für eine Resektion freigelegt sind. Dementsprechend ist es ein Vorteil, wenn Gelenkkopf und Gelenkpfanne unabhängig voneinander eingesetzt werden können und wie ein natürliches Gelenk in Eingriff gebracht werden, ohne dass die oben angesprochene Überdehnung eintritt.

So zeigt die Patentschrift U.S. 4,231,121 ein Fingergelenk, das aus einem Gelenkkopf mit angeformten Schaft und aus einer Gelenkschale mit angeformten Schaft besteht, die in einer Artikulationsstellung zusammenschiebbar sind. Der Bewegungsspielraum geht aus einer Extensionsstellung bis in eine Hyperextension oder bis in eine Artikulationsendstellung. Eine solche Ausführung hat den Nachteil, dass in den Endstellungen beispielsweise in der Hyperextension mit dem Auftreten einer Querkraft ein Abgleiten der Gelenkschale vom Gelenkkopf erfolgen kann, da der Gleichgewichtszustand im wesentlichen davon abhängt, mit welcher Kraft die beiden Gelenkteile von den sie umgebenden Bändern zusammengehalten werden

Aus DE 4 337 922 A1 ist eine Fingergelenkprothese bekannt, von denen Gelenkschale ein Zapfen absbeht, der an seinem freien Ende eine Kugelträgt mit der eine in der Gelenkschale gelagende Teilkugel verriegelad hintergriffen wird.

Aufgabe der Erfindung ist es, künstliche Fingergelenke in dieser Hinsicht zu verbessern. Diese Aufgabe wird dadurch gelöst, dass zwischen Gelenkschale und Gelenkkopf ein zweiter Anschlag in der Hyperextensionsstellung vorhanden ist, der ein Kippen von Führungsstift und Schaft der Gelenkschale um den ersten Anschlag verhindert.

Die Erfindung hat den Vorteil, dass durch das Anbringen eines zweiten Anschlags in einer Stellung, in der ein erster Anschlag für die Hyperextensionsstellung erreicht wird und ein Momentanzentrum für eine Fortsetzung der Drehung bilden würde, die Bewegung gestoppt wird.

Weitere vorteilhafte Verbesserungen ergeben sich aus den abhängigen Ansprüchen 2 bis 12.

So ist es ein Vorteil, dass durch eine entsprechende Ausgestaltung der Tasche Schwenkwinkel α von 80° bis 130° zwischen Hyperextensionsstellung und Artikulationsendstellung möglich sind, die im Schwenkbereich eines natürlichen Fingergelenks liegen.

Eine Ausführungsform sieht eine in Richtung der Hyperextensionsstellung an der Gelenkschale angeformte Kappe vor, welche bei Erreichen der Hyperextensionsstellung auf einen zweiten Anschlag am Gelenkkopf auftrifft, der über einen Drehpunkt am Gelenkkopf hinaus um eine Distanz S zu dessen Schaft hin verschoben ist. Ein Vorteil dieser Kappe ist, dass sie bestimmte Bänder auf Distanz hält und gleichzeitig durch diese Bänder geführt ist. Der Führungsstift kann als runder Stift aus der Gelenkschale vorstehen, was herstelltechnisch einfach ist. Ein weiterer Vorteil dieser Kappe besteht darin, dass bei einer Greifbewegung die palmare Luxation unterbunden wird.

Eine weitere Ausführungsform sieht vor, dass die Tasche über einen Drehpunkt hinaus eine hinterschnittene Erweiterung mit einem zweiten Anschlag aufweist, der entgegengesetzt zum ersten Anschlag angeordnet ist, um den über den Drehpunkt hinaus verlängerten Führungsstift aufzuhalten, wenn er am ersten Anschlag auftrifft. Diese Ausgestaltung hat den Vorteil, dass der zweite Anschlag innerhalb des Gelenkkopfes liegt und äusserlich nicht stören kann.

Zwischen Führungsstift und Tasche kann ein Spiel zu den seitlichen Führungswangen vorgesehen sein, das mehr als 5 % der Breite des Führungsstiftes beträgt, um eine Artikulation in einer seitlich leicht abgewinkelten Position der Schaftachsen zu ermöglichen. Dies hat den Vorteil, dass Finger trotz der Spreizstellung der Mittelhandknochen parallel zueinander artikulieren können.

Bei einem Rechteckquerschnitt des Führungsstiftes kann das Spiel zu den Führungswangen so abgestimmt sein, dass durch eine Rechteckdiagonale, die grösser als der Abstand der Führungswangen ist, eine Verdrehsicherung erreicht wird. Ein um das Spiel zu den Führungswangen reduzierter Führungsstift hat den Vorteil, dass die Freiheitsgrade für die Gelenkbewegungen gleich wie bei einem natürlichen Fingergelenk vorhanden sind.

Ein weiterer Vorteil besteht darin, dass die Lagerflächen von Gelenkkopf und Lagerschale in der Hyperextensionsstellung kongruent sind, um in dieser Stellung die grössten Lagerkräfte aufnehmen zu können, dass die Krümmung des Gelenkkopfes in der Artikulationsrichtung jedoch zunehmen darf, um die Bänder während der Artikulation nicht zu stark zu spannen.

Analog zur Beschränkung der Bewegung in der Hyperextension lässt sich in der Artikulationsendstellung ein dritter Anschlag anbringen und ein Kippen um diesen dritten Anschlag durch einen vierten Anschlag verhindern, der beispielsweise ebenfalls in der Tasche angebracht ist und auf den Führungsstift wirkt.

Um herstelltechnisch eine Tasche mit hinterschnittenen Anschlägen auf einfache Art zu realisieren, kann zunächst eine Tasche ohne Hinterschneidungen hergestellt werden, in der nachträglich angebrachte Bolzen die Hinterschneidungen und Anschläge bilden. Gelenkkopf und Gelenkschale sind mit Vorteil aus körperverträglichen Metallen beispielsweise aus Titan oder Titanlegierungen oder Kobalt-Chrom-Molybdänlegierungen hergestellt. Die Reibungsverhältnisse zwischen den Artikulationsflächen können verbessert werden, wenn eine der Flächen aus Kunststoff beispielsweise aus Polyethylen oder PEAK (Polyaryletherketone) besteht.

Im folgenden wird die Erfindung anhand von Ausführungsbeispielen dargestellt. Es zeigen:
- Fig. 1: Schematisch einen stark vergrösserten Längsschnitt eines erfindungsgemässen künstlichen Fingergelenks in Hyperextensionsstellung;
- Fig. 2: schematisch eine Seitenansicht des Gelenkkopfes von Figur 1;
- Fig. 3: schematisch eine Ansicht auf die Stirnseite des Gelenkkopfes von Figur 2 mit einem rechteckigen in die Tasche hineinragenden Führungsstift;
- Fig. 4: schematisch einen Längsschnitt analog zu Figur 1, bei welchem Anschläge für einen Führungsstift durch nachträglich eingesetzte Bolzen gebildet werden;
- Fig. 5: schematisch eine Seitenansicht des Gelenkkopfes von Figur 4;
- Fig. 6: schematisch eine Ansicht auf die Stirnseite des Gelenkkopfes von Figur 5;
- Fig. 7: schematisch eine weitere Lösung analog zu Figur 1, bei welcher der Gelenkkopf als Spritzteil mit einer Formtrennung senkrecht zur Hyperextension herstellbar ist;
- Fig. 8: schematisch eine Ansicht auf die Stirnseite des Gelenkkopfes von Figur 7;
- Fig. 9: schematisch eine Seitenansicht von Gelenkschale und Führungsstift aus Figur 8;
- Fig. 10: schematisch einen Längsschnitt durch eine weitere Ausführung eines künstlichen Fingergelenks mit einer an der Gelenkschale angeformten Kappe;
- Fig. 11: schematisch eine Ansicht des Gelenkkopfes von Figur 10;
- Fig. 12: schematisch eine als Kunststoffteil hergestellte Lagerfläche, die als Lagerschale an der Gelenkschale von Figur 10 aufsteckbar ist;
- Fig. 13: schematisch die Gelenkschale von Figur 10 mit reduzierten Dimensionen für die Aufnahme des Kunststoffteils in Figur 12; und
- Fig. 14: schematisch eine Seitenansicht der Gelenkschale von Figur 10.

Die Figuren der Ausführungsbeispiele zeigen ein künstliches Fingergelenk mit einem konvexen Gelenkkopf 1 und mit einer konkaven Gelenkschale 2, die unabhängig voneinander jeweils mit einem Schaft 3, 4 an einem Knochenende 5, 6 befestigbar sind und in einer Artikulationsebene aus einer Extensionsstellung 7 mit parallelen Schaftachsen 8, 9 in eine Hyperextensionsstellung 10 oder in eine Artikulationsendstellung 11 bewegbar sind. Aus der Gelenkschale 2 steht ein Führungsstift 12 in der Richtung ihrer Schaftachse 9 vor und ragt in eine Tasche 13 des Gelenkkopfes 1 hinein, wobei die Tasche 13 einen ersten Anschlag 16 für den Führungsstift 12 in der Hyperextensionsstellung 10 aufweist. Ein zweiter Anschlag 17 zwischen Gelenkschale 2 und Gelenkkopf verhindert in der Hyperextensionsstellung 10 ein Kippen von Führungsstift 12 und Schaft 4 der Gelenkschale 2 um den ersten Anschlag 16.

Nachfolgend werden gleiche Hinweiszeichen für gleiche Funktionen verwendet.

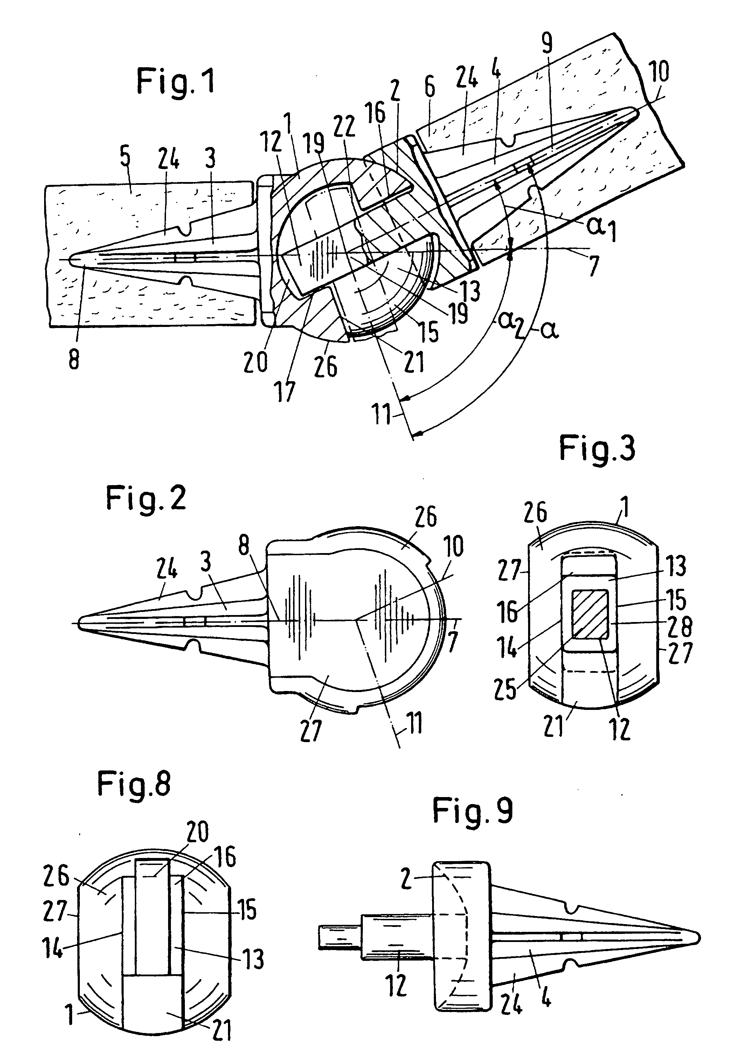

Im Ausführungsbeispiel der Figuren 1, 2 und 3 ist der Gelenkkopf 1 mit einem Schaft 3 in einem Knochenende 5 verankert. Die Verankerung wird durch Rippen 24 am Schaft 3 unterstützt. Der Gelenkkopf 1 ist mit einer kugelförmigen Lagerfläche 26 versehen, deren Mittelpunkt 19 einen Drehpunkt auf der Schaftachse 8 bildet. Die Seitenflächen des Gelenkkopfes 1 weisen eine Abflachung 27 auf. In den Gelenkkopf 1 ist eine Tasche 13 mit seitlichen Führungswangen 14, 15 eingearbeitet. In der Hyperextensionsstellung 10 der Gelenkschale 2 bildet die Tasche 13 einen ersten Anschlag 16 für den aus der Gelenkschale 2 in ihrer Schaftachse 9 vorstehenden Führungsstift 12 und in der Artikulationsendstellung 11 einen dritten Anschlag 21 für den Führungsstift 12. Die Tasche 13 ist hinterschnitten und weist eine Erweiterung 20 auf. Innerhalb dieser Erweiterung 20 kann der über den Drehpunkt 19 verlängerte Führungsstift 12 mitdrehen und trifft in der Hyperextensionsstellung 10 auf einen zweiten Anschlag 17, der um 180° zum ersten Anschlag 16 gedreht ist und ein Kippen von Führungsstift 12 und seinem zugehörigen Schaft 4 um den ersten Anschlag 16 verhindert. Ähnlich ist die Situation in der Artikulationsendstellung, in der ein vierter Anschlag 22 ein Kippen um den dritten Anschlag 21 verhindert. Die Gelenkschale 2 dreht mit ihrer konkaven Lagerschale um den Mittelpunkt 19. Sie ist ebenfalls mit einem Schaft 4 und mit Rippen 24 in einem Knochenende 6 verankert. Die Gelenkschale kann aus einer Extensionsstellung, in der beide Schaftachsen in ihrer Projektion (Figur 1) miteinander fluchten um einen Teilwinkel α₁ in eine Hyperextensionsstellung 10 oder um einen Teilwinkel α₂ in eine Artikulationsendstellung 11 bewegt werden, wobei die Bewegung jeweils durch zwei zusammenwirkende Anschläge 16, 17; 21, 22 begrenzt wird. Der Schwenkwinkel α des Gelenks entspricht der Summe von α₁ und α₂ und beträgt zum Beispiel 110°.

In Figur 3 ist der Querschnitt 25 von einem rechteckigen Führungsstift 12 eingezeichnet, der ein Gesamtspiel 28 zu den Führungswangen 14, 15 der Tasche 13 aufweist, welches 40 % der Schaftdicke entspricht. Trotzdem verhindert der Rechteckquerschnitt mit einer Diagonalen, die grösser als der Abstand der Führungswangen 14, 15 ist, eine extreme Drehung des Führungsstiftes 12 und seines zugehörigen Schaftes 4 um die Schaftachse 9.

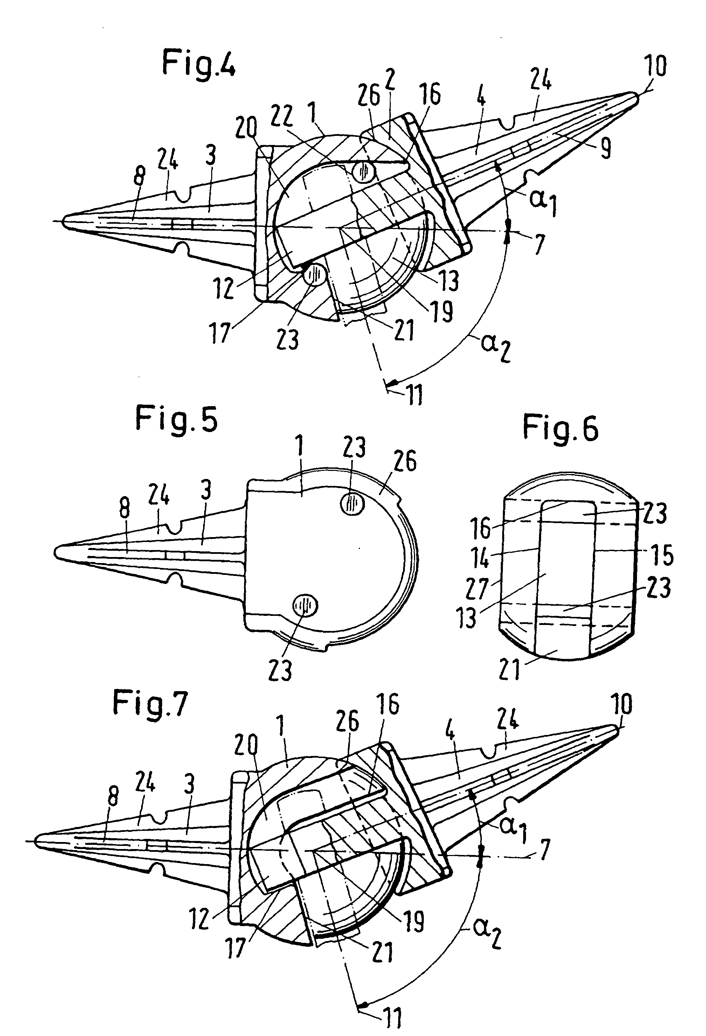

Die Erweiterung 20 der Tasche 13 kann herstelltechnisch auf verschiedene Weise vorgenommen werden. Bei einem Führungsstift 12 mit rundem Querschnitt kann die Erweiterung 20 vorgebohrt und anschliessend mit einem Fingerfräser fertig bearbeitet werden. Eine andere Möglichkeit besteht darin, eine Führungswange 14 erst nach dem Herstellen der Erweiterung 20 am Gelenkkopf 1 zu befestigen. Eine weitere Möglichkeit ist in den Figuren 4, 5 und 6 aufgeführt.

In Figur 4, 5 und 6 wird der Gelenkkopf 1 als Spritzteil geplant. Schaft 3, Rippen 24 und Gelenkkopf 1 sind einteilig vorgesehen. Die Tasche 13 und ihre Erweiterung 20 sind ohne Hinterschneidung für eine Entformung in der Richtung der Schaftachse 8 dimensioniert. Der erste Anschlag 16 und der dritte Anschlag 21 werden durch Begrenzungen der Tasche 13 gebildet. Der zweite Anschlag 17 für die Hyperextensionsstellung 10 wird durch einen nachträglich eingesetzten Bolzen 23 gebildet. Ebenso wird der vierte Anschlag 22 für die Artikulationsendstellung 11 durch einen nachträglich eingesetzten Bolzen 23 gebildet. Bei zwei Bolzen 23 könnte man grundsätzlich alle Anschläge 16, 21, 17, 22 mit den Bolzen realisieren; allerdings wird dann der Hebelarm für ein aufzunehmendes Moment kürzer. Die Gelenkschale 2 mit Führungsstift 12 kann gleich wie in Figur 1 ausgeführt sein.

In den Figuren 7, 8 und 9 wird für den Gelenkkopf 1 ein Spritzteil ohne Bolzen vorgeschlagen, wobei ein vierter Anschlag wegfällt. Eine schlitzförmige Erweiterung 20 der Tasche 13 bildet in der Richtung der Extension 7 einen Hinterschnitt mit einem zweiten Anschlag 17 für die Hyperextensionsstellung 10, wobei eine Entformung in der Richtung der Hyperextension möglich ist. Der über den Drehpunkt 19 vorstehende Führungsstift 12 ist an seiner Spitze in seiner Breite reduziert worden, um in die schlitzförmige Erweiterung 20 einzugreifen und in der Hyperextensionsstellung auf den zweiten Anschlag 17 aufzutreffen, währen der erste Anschlag 16 durch die neben dem Schlitz verbleibenden Schultern für den breiteren Teil des Führungsstiftes 12 gebildet wird. In der normalerweise schwächer belasteten Artikulationsendstellung 11 besteht ein dritter Anschlag 21, der ein Kippen um diesen Anschlag nicht vollständig ausschliesst. Die Befestigungselemente, Schäfte 3, 4 und Rippen 24 sowie die äusseren Dimensionen sind wie in Figur 1 ausgeführt.

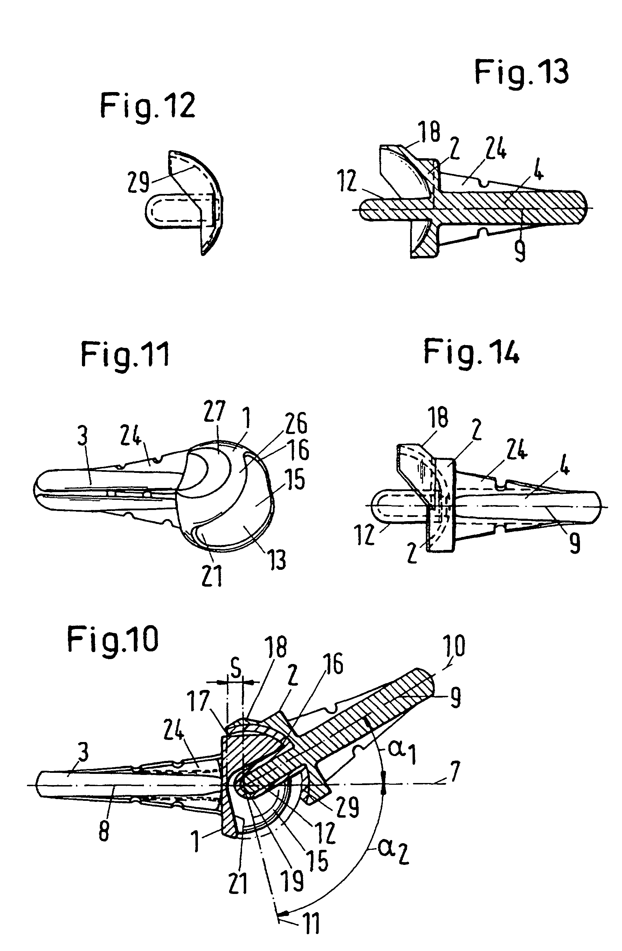

In den Figuren 10 bis 14 ist eine weitere Ausführungsform für ein künstliches Fingergelenk gezeigt. Ein Gelenkkopf 1 ist mit einem Schaft 3 mit Schaftachse 8 und mit Rippen 24 versehen. Er bildet eine kugelige Lagerfläche 26 mit Drehpunkt 19. Die Seitenflächen sind mit Abflachungen 27 versehen. Eine Tasche 13, die von der Hyperextensionsstellung 10 bis zur Artikulationsendstellung 11 reicht, bildet seitliche Führungswangen 15 sowie einen ersten Anschlag 16 und einen dritten Anschlag 21 für einen Führungsstift 12, welcher aus der Gelenkschale 2 in die Tasche 13 hineinragt. Figur 12 zeigt ein Kunststoffspritzteil, welches als Kunststofflagerfläche 29 auf eine Gelenkschale 2 in Figur 13 aufsteckbar ist. Die so zusammengesteckte Gelenkschale 2 (Figur 14) ist mit einem Schaft 4 und Rippen 24 verankerbar. Der Führungsstift 12 steht in der Richtung ihrer Schaftachse 9 vor. An die eigentliche Lagerschale ist eine Kappe 18 in der Richtung der Hyperextension angeformt. In der Hyperextensionsstellung 10 (Fig. 10) von Lagerschale 2 und Gelenkkopf 1 steht diese Kappe 18 in ihrer Projektion auf die Schaftachse 8 des Gelenkkopfes um einen Betrag S über den Drehpunkt 19 vor und trifft an der Aussenkontur des Gelenkkopfes auf einen zweiten Anschlag 17, der ein Kippen um den ersten Anschlag 16 verhindert. Die Gelenkschale 2 kann aus einer Extensionsstellung 7, in der die projizierten Schaftachsen 8, 9 zusammenfallen, um einen Teilwinkel α₁ in die Hyperextensionsstellung 10 oder um einen Teilwinkel α₂ in die Artikulationsendstellung 11 geschwenkt werden. Die Summe der beiden Teilwinkel liegt zwischen 100° und 110°. Analog zu den Abflachungen 27 ist die Kappe 18 seitlich abgeschnitten. Dies hat den Vorteil, dass die Seitenbänder eine Verdrehsicherung um die Schaftachse 9 bilden können.

## Patentansprüche

1. Künstliches Fingergelenk mit einem konvexen Gelenkkopf (1) und mit einer konkaven Gelenkschale, die unabhängig voneinander jeweils mit einem Schaft (3, 4) in einem Knochenende (5, 6) befestigbar sind und in einer Artikulationsebene aus einer Extensionsstellung (7) mit parallelen Schaftachsen (8, 9) in eine Hyperextensionsstellung (10) oder in eine Artikulationsendstellung (11) bewegbar sind,
wobei aus der Gelenkschale (2) ein Führungsstift (12) in der Richtung ihrer Schaftachse (9) vorsteht, welcher in eine Tasche (13) des Gelenkkopfes (1) hineinragt, wobei die Tasche (13) einen ersten Anschlag (16) für den Führungsstift (12) in der Hyperextensionsstellung (10) aufweist, **dadurch gekennzeichnet, dass** zwischen Gelenkschale (2) und Gelenkkopf (1) ein zweiter Anschlag (17) in der Hyperextensionsstellung (10) vorhanden ist, der ein Kippen von Führungsstift (12) und Schaft (4) der Gelenkschale (2) um den ersten Anschlag (16) verhindert.

2. Künstliches Fingergelenk nach Anspruch 1, **dadurch gekennzeichnet, dass** die Tasche (13) zwischen der Hyperextensionsstellung (10) und der Artikulationsendstellung (11) einen Schwenkwinkel α zwischen 80° und 130° zulässt.

3. Künstliches Fingergelenk nach einem der Ansprüche 1 oder 2, **dadurch gekennzeichnet, dass** die konkave Gelenkschale (2) in Richtung der Hyperextensionsstellung eine angeformte Kappe (18) aufweist, welche bei Erreichen der Hyperextensionsstellung (10) auf dem zweiten Anschlag (17) am Gelenkkopf auftrifft, der von einem Drehpunkt (19) im Gelenkkopf um eine Distanz S zu dessen Schaft (3) hin verschoben ist.

4. Künstliches Fingergelenk nach einem der Ansprüche 1 oder 2, **dadurch gekennzeichnet, dass** die Tasche (13) über einen Drehpunkt (19) hinaus eine hinterschnittene Erweiterung (20) mit dem zweiten Anschlag (17) aufweist, der entgegengesetzt zum ersten Anschlag (16) angeordnet ist, und dass der Führungsstift (12) über den Drehpunkt (19) hinaus in die hinterschnittene Erweiterung (20) hineinragt.

5. Künstliches Fingergelenk nach einem der Ansprüche 1 bis 4, **dadurch gekennzeichnet, dass** der Führungsstift (12) einen runden Querschnitt und ein seitliches Gesamtspiel (28) von mehr als 5 % seines Durchmessers zu Führungswangen (14, 15) der Tasche (13) aufweist.

6. Künstliches Fingergelenk nach einem der Ansprüche 1 bis 4, **dadurch gekennzeichnet, dass** der Führungsstift (12) einen Rechteckquerschnitt (25) aufweist und ein seitliches Gesamtspiel (28) von mehr als 5 % seiner Breite zu Führungswangen (14, 15) der Tasche (13) aufweist.

7. Künstliches Fingergelenk nach Anspruch 6, **dadurch gekennzeichnet, dass** der Rechteckquerschnitt eine Diagonale aufweist, die grösser als der Abstand der Führungswangen (14, 15) ist, um eine Verdrehsicherung zu erreichen.

8. Künstliches Fingergelenk nach einem der Ansprüche 1 bis 7, **dadurch gekennzeichnet, dass** die konvexe Lagerfläche des Gelenkkopfes (1) und die konkave Lagerfläche der Gelenkschale (2) in der Hyperextensionsstellung kongruent sind und dass die Krümmung der konvexen Lagerfläche des Gelenkkopfes (1) in der Richtung der Artikulation zunimmt.

9. Künstliches Fingergelenk nach einem der Ansprüche 1 bis 8, **dadurch gekennzeichnet, dass** ein dritter Anschlag (21) in der Tasche (13) die Bewegung des Führungsstiftes (12) in der Artikulationsendstellung (11) einschränkt und dass ein vierter Anschlag (22) in der Tasche (13) vorhanden ist, der ein Kippen von Führungsstift (12) und seinem Schaft (4) um den dritten Anschlag (21) verhindert.

10. Künstliches Fingergelenk nach einem der Ansprüche 1 bis 9, **dadurch gekennzeichnet, dass** Anschläge (16, 17b, 21, 22) als nachträglich in der Tasche (13) angebrachte Bolzen (23) ausgeführt sind.

11. Künstliches Fingergelenk nach einem der Ansprüche 1 bis 10, **dadurch gekennzeichnet, dass** Gelenkkopf (1) und Gelenkschale (2) mit ihren Schäften (3, 4) aus Metall bestehen.

12. Künstliches Fingergelenk nach einem der Ansprüche 1 bis 11, **dadurch gekennzeichnet, dass** Gelenkkopf (1) oder Gelenkschale (2) Artikulationsflächen aufweisen die aus Kunststoff (29) bestehen.

## Claims

1. Artificial finger joint having a convex joint head (1) and a concave joint shell which can be fastened independently of one another with a respective shaft (3, 4) in a bone end (5, 6) and which can be moved in an articulation plane from an extension position (7) with parallel shaft axes (8, 9) into a hyperextension position (10) or into an articulation end position (11), wherein a guide pin (12) projects out of the joint shell (2) in the direction of the shaft axis (9) of the joint shell and protrudes into a pocket (13) of the joint head (1), with the pocket (13) having a first abutment (16) for the guide pin (12) in the hyperextension position (10), **characterized in that** a second abutment (17) is present between the joint shell (2) and the joint head (1) in the hyperextension position (10) and prevents a tilting of the guide pin (12) and of the shaft (4) of the joint shell (2) about the first abutment (16).

2. Artificial finger joint in accordance with claim 1, **characterized in that** the pocket (13) permits a pivotal angle α between 80° and 130° between the hyperextension position (10) and the articulation end position (11).

3. Artificial finger joint in accordance with any one of the claims 1 or 2, **characterized in that** the concave joint shell (2) has a molded on cap (18) in the direction of the hyperextension position which, when the hyperextension position (10) is reached, encounters the second abutment (17) at the joint head which is displaced by a distance S from a point of rotation (19) in the joint head in the direction towards the shaft (3) of the latter.

4. Artificial finger joint in accordance with any one of the claims 1 or 2, **characterized in that**, beyond a point of rotation (19), the pocket (13) has an undercut extension (20) with the second abutment (17) which is arranged oppositely to the first abutment (16); and **in that** the guide pin (12) protrudes outwardly beyond the point of rotation (19) into the undercut extension (20).

5. Artificial finger joint in accordance with any one of the claims 1 to 4, **characterized in that** the guide pin (12) has a round cross-section and a total lateral clearance (28) of more than 5 % of its diameter from guide cheeks (14, 15) of the pocket (13).

6. Artificial finger joint in accordance with any one of the claims 1 to 4, **characterized in that** the guide pin (12) has a rectangular cross-section (25) and has a total lateral clearance (28) of more than 5 % of its width from guide cheeks (14, 15) of the pocket ( 13).

7. Artificial finger joint in accordance with claim 6, **characterized in that** the rectangular cross-section has a diagonal which is longer than the spacing of the guide cheeks (14, 15) in order to achieve a rotational security.

8. Artificial finger joint in accordance with any one of the claims 1 to 7, **characterized in that** the convex bearing surface of the joint head (1) and the concave bearing surface of the joint shell (2) are congruent in the hyperextension position; and **in that** the curvature of the convex bearing surface of the joint head (1) increases in the direction of the articulation.

9. Artificial finger joint in accordance with any one of the claims 1 to 8, **characterized in that** a third abutment (21) in the pocket (13) restricts the movement of the guide pin (12) in the articulation end position (11); and **in that** a fourth abutment (22) is present in the pocket (13) which prevents a tilting of the guide pin (12) and its shaft (4) about the third abutment (21).

10. Artificial finger joint in accordance with any one of the claims 1 to 9, **characterized in that** abutments (16, 17b, 21, 22) are formed as pins (23) which are subsequently secured in the pocket (13).

11. Artificial finger joint in accordance with any one of the claims 1 to 10, **characterized in that** the joint head (1) and the joint shell (2) with their shafts (3, 4) consist of metal.

12. Artificial finger joint in accordance with any one of the claims 1 to 11, **characterized in that** the joint head (1) or the joint shell (2) have articulation surfaces which consist of plastic (29).

## Revendications

1. Articulation artificielle du doigt, comportant une tête convexe d'articulation (1) et une capsule concave d'articulation, qui peuvent être fixées chacune, indépendamment l'une de l'autre, par une tige (3, 4) dans une extrémité d'os (5, 6) et qui sont mobiles, dans un plan d'articulation, à partir d'une position d'extension (7), dans laquelle les axes (8, 9) des tiges sont parallèles, pour prendre une position d'hyperextension (10) ou une position finale d'articulation (11), articulation dans laquelle un ergot de guidage (12) fait saillie à partir de la capsule d'articulation (2) dans le sens de l'axe de tige (9) de celle-ci et pénètre dans une poche (13) ménagée dans la tête d'articulation (1), la poche (13) présentant une première butée (16) pour l'ergot de guidage (12) dans la position d'hyperextension (10), **caractérisée en ce qu'**il existe, entre la capsule d'articulation (2) et la tête d'articulation (1), une deuxième butée (17) dans la position d'hyperextension (10) pour empêcher un basculement de l'ergot de guidage (12) et de la tige (4) de la capsule d'articulation (2) autour de la première butée (16).

2. Articulation artificielle du doigt selon la revendication 1, **caractérisée en ce que** la poche (13) autorise, entre la position d'hyperextension (10) et la position finale d'articulation (11), un angle de pivotement α entre 80° et 130°.

3. Articulation artificielle du doigt selon la revendication 1 ou 2, **caractérisée en ce que** la capsule concave d'articulation (2) présente, dans la direction de la position d'hyperextension, une crête (18) qui est formée d'un seul tenant avec elle et qui, lorsque la position d'hyperextension (10) est atteinte, vient s'appliquer sur la deuxième butée (17) présente au niveau de la tête d'articulation, cette butée étant décalée, par rapport à un centre de rotation (19) situé dans la tête d'articulation, d'une distance S vers la tige (3) de la tête.

4. Articulation artificielle du doigt selon la revendication 1 ou 2, **caractérisée en ce que** la poche (13) présente, au-delà d'un centre de rotation (19), un élargissement en contre-dépouille (20) avec la deuxième butée (17), qui est disposée à l'opposé de la première butée (16), et **en ce que** l'ergot de guidage (12) pénètre, au-delà du centre de rotation (19), dans l'élargissement en contre-dépouille (20).

5. Articulation artificielle du doigt selon l'une des revendications 1 à 4, **caractérisée en ce que** l'ergot de guidage (12) présente une section transversale ronde et un jeu latéral total (28), qui représente plus de 5 % de son diamètre, avec les faces de guidage (14, 15) de la poche (13).

6. Articulation artificielle du doigt selon l'une des revendications 1 à 4, **caractérisée en ce que** l'ergot de guidage (12) présente une section transversale rectangulaire (25) et un jeu latéral total (28), qui représente plus de 5 % de sa largeur, avec les faces de guidage (14, 15) de la poche (13).

7. Articulation artificielle du doigt selon la revendication 6, **caractérisée en ce que** la section transversale rectangulaire présente une diagonale, qui est plus grande que l'écartement des faces de guidage (14, 15), afin d'obtenir une sécurité antitorsion.

8. Articulation artificielle du doigt selon l'une des revendications 1 à 7, **caractérisée en ce que** la surface convexe d'appui de la tête d'articulation (1) et la surface concave d'appui de la capsule d'articulation (2) sont congruentes dans la position d'hyperextension, et **en ce que** la courbure de la surface convexe d'appui de la tête d'articulation (1) augmente dans la direction de l'articulation.

9. Articulation artificielle du doigt selon l'une des revendications 1 à 8, **caractérisée en ce qu'**une troisième butée (21) limite, dans la poche (13), le mouvement de l'ergot de guidage (12) dans la position finale d'articulation (11), et **en ce qu'**une quatrième butée (22) est prévue dans la poche (13) pour empêcher un basculement de l'ergot de guidage (12) et de sa tige (4) autour de la troisième butée (21).

10. Articulation artificielle du doigt selon l'une des revendications 1 à 9, **caractérisée en ce que** des butées (16, 17b, 21, 22) sont réalisées sous la forme de boulons (23) fixés par la suite dans la poche (13).

11. Articulation artificielle du doigt selon l'une des revendications 1 à 10, **caractérisée en ce que** la tête d'articulation (1) et la capsule d'articulation (2) sont fabriquées, ainsi que leurs tiges (3, 4) en un métal.

12. Articulation artificielle du doigt selon l'une des revendications 1 à 11, **caractérisée en ce que** la tête d'articulation (1) ou la capsule d'articulation (2) présente des surfaces d'articulation, qui consistent en une matière plastique (29).
